# EUROPEAN PATENT APPLICATION

(11) **EP 4 219 756 A1**
(43) Date of publication of application: **02.08.2023**
(21) Application number: 23152683.1
(22) Date of filing: 20.01.2023
(51) Int. Cl.: C12Q 1/6853, C12Q 1/6867, C12N 15/11

(54) **SYNTHETIC RNA FRAGMENT AND ITS USES FOR RNA-DEPENDENT AMPLIFICATION**

(30) Priority: 24.01.2022 US 202263302163 P; 05.05.2022 US 202263338881 P
(71) Applicant: Mello Biotech Taiwan Co., Ltd., Taipei City 115 (TW)
(72) Inventor: LIN, Shi-Lung, Acadia (US); LIN, Sam, Acadia (US); WU, David TS, Taipei City (TW)
(74) Representative: Lang, Christian

(57) **Abstract**

Disclosed herein is directed to a synthetic ribonucleic acid (RNA) fragment that includes an RNA template flanked by at least of 5'-end RNA-dependent RNA polymerase (RdRp) binding site and a 3'-end RdRp binding site, thus the synthetic RNA fragment can be amplified *in vitro* via an RNA-dependent RNA cycling reaction (RCR). Also disclosed herein is a method for producing an amplified RNA product by use of the present synthetic RNA fragment.

## Description

### CROSS-REFERENCES TO RELATED APPLICATIONS

This application claims priority and the benefit of U.S. Provisional Patent Applications No. 63/302,163, filed January 24, 2022, and No. 63/338,881, filed May 5, 2022, the entireties of which is incorporated herein by reference.

### BACKGROUND OF THE INVENTION

### 1. FIELD OF THE INVENTION

The present disclosure relates to the field of nucleic acid amplification technology. More particularly, the disclosed invention relates to a novel synthetic ribonucleic acid (RNA) fragment and its uses in an RNA-dependent RNA cycling reaction (RCR).

### 2. DESCRIPTION OF RELATED ART

Since initially derived from alphavirus genomes, self-amplifying ribonucleic acids (saRNAs or samRNAs) have been studied and used for developing vaccines against cancers and infectious diseases for decades. The early saRNA design is based on viral replicons that use parts of the viral genome as a backbone to reconstitute a recombinant, self-replicable mRNA platform for gene expression. As a result, saRNA constructs not only contain the basic elements of messenger RNA (mRNA) including a cap, 5'-untranslated region (UTR), a peptide/protein-coding sequence, 3'UTR, and poly(A) tail of variable lengths, but also comprise a viral 19-nucleotide (nt) conserved sequence element (3'CSE) and an alphavirus replicase genes encoding an RNA-dependent RNA polymerase (RdRp) complex, which enables self-transcription and amplification of RNA transcripts *in situ* by recognizing the 3'CSE sequence. As a result of their self-replicative activity, saRNAs can be delivered at a low concentration to achieve comparable antigen expression. Hence, saRNAs are emerging as important RNA vaccine candidates.

The current methodology for producing an amplified RNA product of a desired RNA sequence *in vitro* relies on a method that combines polymerase chain reaction (PCR) and *in vitro* transcription (IVT). Specifically, the method mainly comprises following steps: preparing a reverse-transcribed double-stranded cDNA template from the desired RNA sequence; synthesizing an RNA template via *in vitro* transcription from the reverse-transcribed double-stranded cDNA template; reversely transcribing the synthesized RNA template to form a RNA-cDNA hybrid; amplifying the RNA-cDNA hybrid into promoter-linked double-stranded cDNA by repeating PCR; synthesizing RNA via *in vitro* transcription from the promoter-linked double-stranded cDNA; and digesting deoxyribonucleotides of the amplified hybrid RNA-cDNA products by endonucleases, thereby producing the amplified RNA product.

However, the said method has limitations in amplification of saRNAs *in vitro.* Though the afore-mentioned RdRp can be developed and involved in an RNA-mediated amplification to skip the step of generating RNA-cDNA hybrids, yet the 3'CSE, serving as a RdRp binding side, is too long to be used as potential primers in either general PCR or revers transcription-PCR. Also, because 3'-CSE is a highly structured sequence that easily forms stem-loops, it hinders the RNA transcription from using conventional prokaryotic RNA polymerases, resulting a failure of performing conventional *in vitro* transcription (IVT) method.

In view of the foregoing, there exists in the related art a need for developing RdRp-binding sites capable of combining with the RNA template and serving as promoters for PCR, thereby achieving amplification of saRNAs *in vitro.*

### SUMMARY

The following presents a simplified summary of the disclosure in order to provide a basic understanding to the reader. This summary is not an extensive overview of the disclosure and it does not identify key/critical elements of the present invention or delineate the scope of the present invention. Its sole purpose is to present some concepts disclosed herein in a simplified form as a prelude to the more detailed description that is presented later.

As embodied and broadly described herein, one aspect of the present disclosure is directed to a synthetic ribonucleic acid (RNA) fragment, which comprises an RNA template flanked by a 5'-end RNA-dependent RNA polymerase (RdRp) binding site, a 3'-end RdRp binding site, or a combination thereof, wherein each of the 5'-end and 3'-end RdRp binding sites individually comprises a polyribonucleotide sequence selected from the group consisting of 5'-USUSCYW-3' and 5'-UAGSRVR-3'.

According to some embodiments of the present disclosure, the 5'-end RdRp binding site has a polyribonucleotide sequence of 5'-UCUCCUA-3', 5'-UGUGCUA-3', or 5'- UCUCCCU-3'.

According to some embodiments of the present disclosure, the 3'-end RdRp binding site has a polyribonucleotide sequence of 5'-UAGGAGA-3', 5'-UAGCACA-3', or 5'-UAGGGAGA-3'.

According to some embodiments of the present disclosure, the RNA template comprises a polyribonucleotide sequence or a hybrid of polyribonucleotide and polydeoxyribonucleotide sequence, wherein the polyribonucleotide sequence is a coding RNA or a non-coding RNA.

According to one embodiment of the present disclosure, the coding RNA may be a messenger RNA (mRNA) that encodes an antigen. Specifically, the antigen may be a cancer antigen, a tumor antigen, a bacterial antigen, a viral antigen, a fungal antigen, a parasitic antigen, or a combination thereof.

Example of the tumor antigen includes, but is not limited to, a neoantigen, a tumor-derived lysate, an alpha-fetoprotein (AFP), a carcinoembryonic antigen (CEA), a mucin protein, an epithelial tumor antigen (ETA), a tyrosinase, a melanoma-associated antigen (MAGE), a RAS protein, and a tumor suppressor protein.

The exemplary bacterial antigen may be derived from a bacterial species including *Actinomyces, Aeromonas, Arthrobacter, Bacillus, Bacteroides, Bordetella, Borrelia, Brucella, Campylobacter, Chlamydia, Citrobacter, Clostridium, Corynebacterium, Escherichia, Enterobacter, Gardnerella, Helicobacter, Haemophilus, Klebsiella, Legionella, Listeria, Mycobacterium, Neisseria, Nocardia, Pasteurella, Proteus, Pseudomonas, Ureaplasma, Salmonella, Shigella, Spirillum, Spirochaeta, Staphylococcus, Streptobacillus, Streptococcus, Streptomyces, Treponema,* and *Yersinia;* but is not limited thereto.

The exemplary viral antigen may be derived from a viral species that includes, but is not limited to, *Adenovirus, Alphacoronavirus, Betacoronavirus, Cytomegalovirus, Deltainfluenzavirus, Deltacoronavirus, Gammacoronavirus, Hepacivirus, Hepatovirus, Influenza A virus, Influenza B virus, Influenza C virus, Influenza D virus, Lentivirus, Letovirus, Lymphocryptovirus, Orthopneumovirus, Orthopoxvirus, Papillomavirus, Quaranjavirus, Rotavirus, Simplexvirus,* and *Varicellovirus.*

According to one preferred embodiment of the present disclosure, the viral antigen is derived from a spike protein of *Betacoronavirus.*

The exemplary fungal antigen may be derived from a fungal species that causes a fungal infection, which includes aspergillosis, blastomycosis, candidiasis, chromoblastomycosis, cryptococcosis, histoplasmosis, mycetoma, paracoccidioidomycosis, ringworm and tinea versicolor; yet is not limited thereto.

The exemplary parasitic antigen may be derived from a parasite species that causes a parasitic infection, which includes but is not limited to, African trypanosomiasis, amebiasis, Chagas disease, echinococcosis, fascioliasis, hookworm disease, hymenolepis, leishmaniasis, neurocysticercosis, onchocerciasis, *Plasmodium* infection, paragonimiasis, *Pneumocystis* pneumonia (PCP), schistosomiasis, trichomoniasis, taeniasis, and trichuriasis.

According to some embodiments of the present disclosure, the non-coding RNA is a small interfering RNA (siRNA), a ribosomal RNA (rRNA), a transfer RNA (tRNA), a microRNA (miRNA), or an aptamer.

In certain embodiment of the present disclosure, the miRNA is a precursor miRNA; in another embodiment, miRNA is a mature miRNA.

According to one working example of the present disclosure, the non-coding RNA is derived from the precursor miRNA-302.

According to some embodiments of the present disclosure, the RNA template comprises a polyribonucleotide sequence encoding a transcript of a nonstructural protein; in one preferred embodiment, the nonstructural protein is an RNA-dependent RNA polymerase (RdRp).

Another aspect of the present disclosure is directed to a method for producing an amplified RNA product *in vitro* comprising amplifying the afore-mentioned synthetic RNA fragment via an RNA cycling reaction (RCR), thereby producing the amplified RNA product transcribed from the RNA template of the said synthetic RNA fragment.

According to some embodiments of the present disclosure, the RNA template of the synthetic RNA fragment comprises a polyribonucleotide sequence or a hybrid of polyribonucleotide and polydeoxyribonucleotide sequence, and the polyribonucleotide sequence encodes a transcript of a nonstructural protein. According to one preferred embodiment of the present disclosure, the nonstructural protein is an RNA-dependent RNA polymerase (RdRp).

According to some embodiments of the present disclosure, the present method further comprises adding a five-prime cap (5'cap) and a poly(A) tail to the amplified RNA product.

In some preferred embodiments of the present disclosure, the amplified RNA product is a self-amplifying RNA (saRNA).

Many of the attendant features and advantages of the present disclosure will becomes better understood with reference to the following detailed description considered in connection with the accompanying drawings.

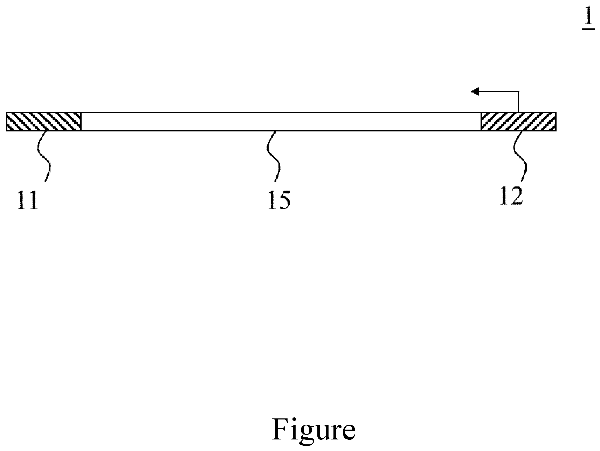

### BRIEF DESCRIPTION OF THE DRAWING

The present description will be better understood from the following detailed description read in light of the accompanying drawing, where:

Figure is a schematic diagram of an exemplary synthetic RNA fragment 1 according to one embodiment of the present disclosure.

In accordance with common practice, the various described features/elements are not drawn to scale but instead are drawn to best illustrate specific features/elements relevant to the present invention. Also, like reference numerals and designations in the various drawings are used to indicate like elements/parts.

### DESCRIPTION

The detailed description provided below in connection with the appended drawings is intended as a description of the present examples and is not intended to represent the only forms in which the present example may be constructed or utilized. The description sets forth the functions of the example and the sequence of steps for constructing and operating the example. However, the same or equivalent functions and sequences may be accomplished by different examples.

### 1. DEFINITIONS

For convenience, certain terms employed in the specification, examples and appended claims are collected here. Unless defined otherwise, all technical and scientific terms used herein have the same meaning as commonly understood by one of the ordinary skill in the art to which this invention belongs.

The singular forms "a", "and", and "the" are used herein to include plural referents unless the context clearly dictates otherwise.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements.

The term "RNA template" as used herein is intended to encompass a desired polyribonucleotide sequence serving as a template for synthesis of copies in RNA-mediated RNA cycling reaction (RCR), by which the desired polyribonucleotide sequence can be amplified. Accordingly, the RNA template may comprise a polyribonucleotide sequence of, or derived from a coding RNA or a non-coding RNA, depending on practical needs. For example, the RNA template may comprise a polyribonucleotide sequence encoding a spike protein of severe acute respiratory syndrome coronavirus 2 (SARS-CoV-2). Alternatively, the RNA template may also comprise a hybrid of polyribonucleotide and polydeoxyribonucleotide sequence, if there are polyribonucleotides in the template.

The term "desired RNA(s)" or "desired polyribonucleotide sequence(s)" interchangeably used herein refers to those RNA fragments or polyribonucleotide sequences that are deemed as targets and expected to be amplified for certain purposes. In the present disclosure, the desired RNA may be a fragment of precursor miRNA, or a transcript of viral nonstructural protein.

The term "RNA-dependent RNA polymerase (RdRp) binding site" as used herein refers to a short RNA sequence (e.g., less than 10 nt) that can be recognized and bound by the RdRp enzyme. Typically, the RdRp binding site can be flanked to the termini of a polynucleotide of DNA or RNA to form a DNA/RNA template useful in amplification methodology, for example, an RNA cycling reaction (RCR). According to the preset disclosure, the RdRp binding site comprises a polyribonucleotide sequence of 5'-USUSCYW-3' or 5'-UAGSRVR-3'.

The term "RNA-dependent" or "RNA-mediated" RNA cycling reaction (RCR)" interchangeably used herein refers to a repeated, cycling reaction that uses polyribonucleotide sequences as templates with the aid of the RNA-dependent RNA polymerases (RdRps) and ribonucleoside triphosphates (rNTPs) as reaction materials, so as to produce amplified RNA.

The term of "nonstructural protein" used herein refers to a protein encoded by a virus but that is not part of the viral particle. The nonstructural protein (nsP or NSP) typically includes various enzymes and transcription factors that the virus uses to replicate itself, such as, a viral protease, an RNA replicase, and/or RNA template-directed polymerases.

The term "microRNA" or "miRNA" used herein refers to a class of non-coding RNAs that play roles in regulating gene expression. Most miRNAs are transcribed from DNA sequences into primary miRNAs (pri-miRNAs) and processed into precursor miRNAs (pre-miRNAs) and finally mature miRNAs. Accordingly, the term miRNA used herein refers to all non-coding RNAs involved in miRNA processing and maturation, preferably including precursor miRNAs and mature miRNAs.

The term "infectious diseases" used herein refers to disorders caused by pathogens, such as bacteria, viruses, fungi or parasites, which typically cause acute symptoms, such as fever, inflammation, upper respiratory symptoms, diarrhea, and the like.

### 2. DETAIL DESCRIPTION OF PREFERRED EMBODIMENTS

The present disclosure is based, at least in part, on the development of short ribonucleotides (less than 10 nt) that can be flanked on two termini of a ribonucleic acid (RNA) template and recognized by an RNA-dependent RNA polymerase (RdRp) to initiate *in situ* transcription. Accordingly, the present disclosure provides a synthetic RNA fragment that comprises an RNA template flanked by novel short ribonucleotides recognizable for RdRp, thus the synthetic RNA fragment can be amplified in vitro via an RNA-dependent RNA cycling reaction (RCR). Also disclosed herein is a method for producing an amplified RNA product by use of the present synthetic RNA fragment.

### 2.1 Synthetic RNA fragment of the present disclosure

One aspect of the present invention is directed to a synthetic ribonucleic acid (RNA) fragment suitable for used in RNA-dependent RNA amplification process. The synthetic RNA fragment comprises an RNA template flanked by a 5'-end RNA-dependent RNA polymerase (RdRp) binding site, a 3'-end RdRp binding site, or a combination thereof, wherein each of the 5'-end and 3'-end RdRp binding sites individually comprises a polyribonucleotide sequence selected from the group consisting of 5'-USUSCYW-3' and 5'-UAGSRVR-3'.

Referring more particularly to the figure, which depicts an exemplary structure of the present synthetic RNA fragment 1 according to one embodiment of the present disclosure. It should be noted that the RNA template 15 can be either flanked by the 5'-end RdRp binding site 11 or the 3'-end RdRp binding site 12, or both. Preferably, the RNA template 15 is flanked by both the 5'-end and 3'-end RdRp binding sites (11, 12), as a schematic structure depicted in the figure.

The synthetic RNA fragment of the present disclosure can be produced via procedures and/or tools well known in the art, including *in vitro* transcribing directly from a DNA or cDNA template, either single-stranded or double-stranded. According to some embodiments of the present disclosure, the synthetic RNA fragment is produced by a method combining a reverse transcription PCR (RT-PCR) or a general PCR procedure and an *in vitro* transcription (IVT) procedure, as described in published patent documents US 2022/0396798 and WO 2022/260718 A1, and a research paper published by Li & Ji, Methods in Molecular Biology, vol. 221, 2003. Specifically, a double-stranded cDNA template of a target gene or a sequence fragment (hereinafter, cDNA template) is produced from an isolated single-stranded DNA or RNA, an RNA-DNA hybrid, or a double-stranded DNA via the conventional RT-PCR with addition of primer pairs (including forward and reverse primers for PCR). According to the present disclosure, the primers pairs are designed and synthesized to complement the sequence of RdRp binding sites; thus, the complementary DNA sequences of the present RdRp binding sites are embedded and incorporated in the 5'- and 3'- ends of the cDNA templates. Note that the present RdRp binding sites serve as a promoter-like motif for initiating RdRp activities. The thus-produced cDNA templates are subjected to the IVT procedure to create synthetic RNA fragments. According to present disclosure, to conduct the IVT procedure, the resulting cDNA template can be cloned into a plasmid or a viral vector via procedures and/or tools well known in the art, thereby producing the present synthetic RNA fragment, which comprises the RNA sequences of the RNA template flanked by the 5'-end and 3'-end RdRp binding sites. In alternative embodiment, the thus produced cDNA template incorporating the primers of RdRp binding sites can serve as a starting material of RNA-dependent RNA cycling reaction (RCR), thereby directly producing the present synthetic RNA fragment.

According to some preferred embodiments of the present disclosure, the 5'-end RdRp binding site has a polyribonucleotide sequence of 5'-UCUCCUA-3', 5'-UGUGCUA-3', or 5'-UCUCCCU-3'. According to other preferred embodiments of the present disclosure, the 3'-end RdRp binding site has a polyribonucleotide sequence of 5'-UAGGAGA-3', 5'-UAGCACA-3', or 5'-UAGGGAGA-3'. In one working example, the RNA template is flanked by 5'-UCUCCUA-3' and 5'-UAGGAGA-3'. In other working example, the RNA template is flanked by 5'-UGUGCUA-3' and 5'-UAGCACA-3'. In a further working example, the RNA template is flanked by 5'-UCUCCCU-3' and 5'-UAGGGAGA-3'.

Alternatively or optionally, each of the 5'-end and 3'-end RdRp binding sites of the present disclosure individually comprises a polyribonucleotide sequence selected from the group consisting of 5'-UAGSRVRA-3' and 5'-UYBYHCUA-3'.

As set forth above, the RNA template of the present disclosure may be, or contain any of desired polyribonucleotide sequences of a coding RNA or a non-coding RNA, as long as it is flanked by two RdRp binding sites so as to form the synthetic RNA fragment useful for producing RNA copies.

According to some embodiments of the present disclosure, the coding RNA includes a messenger RNA (mRNA) that encodes an antigen. Specifically, the coding RNA may be the mRNA encoding a cancer antigen, a tumor antigen, a fungal antigen, a parasitic antigen, a bacterial antigen, a viral antigen, or a combination thereof.

Examples of the afore-mentioned tumor antigen include, but are not limited to, a neoantigen, a tumor-derived lysate, an alpha-fetoprotein (AFP), a carcinoembryonic antigen (CEA), a mucin protein, an epithelial tumor antigen (ETA), a tyrosinase, a melanoma-associated antigen (MAGE), a RAS protein, a tumor suppressor protein, and a combination thereof.

Examples of the afore-mentioned bacterial antigen include those derived from a bacterial species, which includes genera of *Actinomyces, Aeromonas, Arthrobacter, Bacillus, Bacteroides, Bordetella, Borrelia, Brucella, Campylobacter, Chlamydia, Citrobacter, Clostridium, Corynebacterium, Escherichia, Enterobacter, Gardnerella, Helicobacter, Haemophilus, Klebsiella, Legionella, Listeria, Mycobacterium, Neisseria, Nocardia, Pasteurella, Proteus, Pseudomonas, Ureaplasma, Salmonella, Shigella, Spirillum, Spirochaeta, Staphylococcus, Streptobacillus, Streptococcus, Streptomyces, Treponema,* and *Yersinia,* but not limited thereto.

Examples of the afore-mentioned viral antigen include those derived from a viral species, which includes but is not limited to, *Adenovirus, Alphacoronavirus, Betacoronavirus, Cytomegalovirus, Deltainfluenzavirus, Deltacoronavirus, Gammacoronavirus, Hepacivirus, Hepatovirus, Influenza A virus, Influenza B virus, Influenza C virus, Influenza D virus, Lentivirus, Letovirus, Lymphocryptovirus, Orthopneumovirus, Orthopoxvirus, Papillomavirus, Quaranjavirus, Rotavirus, Simplexvirus,* and *Varicellovirus.* In some embodiments, the viral antigen is derived from a *Betacoronavirus* genus; preferably is a spike (S) protein or a nucleocapsid (N) protein of Severe acute respiratory syndrome-related coronavirus (SARS-CoV-2). In alternative embodiments, the viral antigen is derived from a *Hepacivirus* genus; preferably is a core antigen of hepatitis C virus (HCV).

Examples of the fungal antigen include those derived from a fungal species that causes a fungal infection, which includes but is not limited to, aspergillosis, blastomycosis, candidiasis, chromoblastomycosis, cryptococcosis, histoplasmosis, mycetoma, paracoccidioidomycosis, ringworm, tinea versicolor, and a combination thereof.

Examples of the afore-mentioned parasitic antigen include those derived from a parasite species that causes a parasitic infection including but not limited to, African trypanosomiasis, amebiasis, Chagas disease, echinococcosis, fascioliasis, hookworm disease, hymenolepis, leishmaniasis, neurocysticercosis, onchocerciasis, *Plasmodium* infection, paragonimiasis, *Pneumocystis* pneumonia (PCP), schistosomiasis, trichomoniasis, taeniasis, trichuriasis, and a combination thereof.

According to alternative embodiments of the present disclosure, the RNA template contains a polyribonucleotide sequence of the non-coding RNA, such as, small interfering RNA (siRNA), ribosomal RNA (rRNA), transfer RNA (tRNA), microRNA (miRNA), or aptamer. Typically, examples of the miRNA include, but are not limited to, a precursor miRNA or a mature miRNA. According to one working example, the present RNA template contains a polyribonucleotide sequence derived from precursor miRNA-302 cluster of humans.

In some alternative embodiments, in addition to the coding or noncoding RNA sequences, the synthetic RNA fragment further comprises a polyribonucleotide sequence encoding nonstructural proteins (nsPs) derived from viruses. Typically, the transcript of the polyribonucleotide sequence is translated to a functional protein, such as an RNA replicase and/or an RNA-dependent RNA polymerase (RdRp) to initiate the RNA-mediated replication. Examples of the nonstructural protein suitable for use in the present synthetic RNA fragment may be those derived from viral species or genera including but not limited to, *Rotavirus, Betacoronavirus,* Influenza viruses, *Hepacivirus, Orthohepadnavirus, Phlebovirus,* and the like. In one working embodiment of the present disclosure, the transcripts are derived from alphavirus and have four nonstructural proteins (nsP1-4) that are expected to form a whole RdRp protein after translation. In another working embodiment of the present disclosure, the nonstructural proteins are derived from Severe acute respiratory syndrome-related coronaviruses (e.g., SARS-CoV-1 and SARS-CoV-2). In still another working embodiment, the nonstructural proteins are derived from hepatitis C virus (HCV) RdRp, as known as NS5B.

### 2.2 Amplification via use of the present synthetic RNA fragment

Another aspect of the present disclosure is directed to a method for producing an amplified RNA product *in vitro.* The method comprises at least the step of, amplifying the present synthetic RNA fragment via an RNA cycling reaction (RCR), thereby producing the amplified RNA product transcribed from the RNA template of the present synthetic RNA fragment.

According to the present disclosure, the synthetic RNA fragment encompasses at least one RdRp-binding site that can be recognized by RNA replicases (i.e., a RdRp enzyme), therefor can be used in RNA-dependent (or RNA-mediated) RCR. The processes of RNA-dependent RCR have been described in U.S. patent application No. 17/648,336, and U.S. provisional applications No. 63/302,163 and 63/338,881. Briefly, the synthetic RNA fragment and RNA replicases (i.e., a RdRp enzyme) are provided in a buffer condition containing ribonucleoside triphosphate molecules (rNTPs) required for RNA synthesis. When the amplification starts, the synthetic RNA fragment, in most cases a sense-strand RNA sequence, can serve as a template, let the RdRp enzyme binds to the RdRp-binding site and carry out the replication to produce a complementary, antisense-strand RNA sequence. The antisense-strand RNA sequence also encompasses the RNA template and the RdRp-binding site; thus, the antisense-strand RNA sequence can serve as another template for amplifying the sense-strand RNA sequences, which further serve as templates for amplifying antisense-strand RNA sequences again. In other words, both of sense-strand and antisense-strand RNAs can serve as templates for each other to conduct the desired RNA amplification via the RNA-dependent RCR. Accordingly, multiple amplification cycles can be performed under predetermined conditions including temperatures and times according to practical needs, and eventually the synthetic RNA fragment with desired sequences are amplified to several times, such as a 10- to 1000-fold amplification , for example, a 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 fold amplification, thereby obtaining the desired, amplified RNA product. In one working example, the resulting amplified RNA product is a 10-fold increase to the initial synthetic RNA fragment. In another working example, the amplified RNA product is a 100-fold increase to the synthetic RNA fragment. In the other working example, the amplified RNA product has a 1000-fold increase.

According to some embodiments the amplification is conducted with a pH level ranging from 6.0 to 8.0, at 20°C to 45°C for 20 minutes to 6 hours.

According to the present disclosure, the thus-obtained amplified RNA product may be in either a primary, secondary or tertiary structure, depending on the condition of reaction. In some embodiments, the structure of the present amplified RNA product is in a single-stranded, secondary structure conformation. In other embodiments, the present amplified RNA product is mainly composed of double-stranded RNA products.

As stated above, the synthetic RNA fragment used in the RNA-dependent RCR further comprises a polyribonucleotide sequence encoding nonstructural proteins (nsPs) derived from viruses. On the RNA template of the synthetic RNA fragment, the said polyribonucleotide sequence is preferably disposed at the upstream of the desired RNA. In some embodiments of the present disclosure, the transcripts are derived from alphavirus and have four nonstructural proteins (nsP1-4) that are expected to form a whole RdRp protein after translation. In other embodiments of the present disclosure, the nonstructural proteins are derived from Severe acute respiratory syndrome-related coronavirus (e.g., SARS-CoV-1 and SARS-CoV-2). In still another working embodiment, the nonstructural proteins are derived from hepatitis C virus (HCV) RdRp, as known as NS5B.

Accordingly, the amplified RNA producing based on the whole length of the synthetic RNA fragment also encompass the polyribonucleotide sequences encoding nonstructural protein transcripts. The present amplified RNA product is further subjected to RNA processing, including but not limited to RNA splicing, nonsense-mediated decay (NMD), RNA editing, 5'-capping, 3'-poly(A) tailing, and a combination thereof, by the means and tools well-known in the art, such as RNA-guided RNA modification via rRNAs, snRNAs, tRNAs, small nucleolar (sno) RNAs, and perhaps mRNAs. In one preferred embodiment, a five-prime cap (5' cap) and a poly(A) tail are added to the amplified RNA product, thereby producing a mature messenger RNA. Structurally, the thus-produced amplified RNA product contains at least the 5'cap, 5' UTR, at least one nonstructural proteins (nsP), a translation initiation site (TIS), a desire sequence of interest (either coding or non-coding sequences), 3' UTR, and the 3' poly(A) tail. Accordingly, the thus-obtained amplified RNA product possesses a self-amplification property, therefore can serve as a self-amplifying RNA (saRNA) for versatile application.

Taken together, the present synthetic RNA fragment structurally contains 5'-end and 3'-end RdRp binding sites that required for RCR-mediated RNA amplification, and a polyribonucleotide sequences of nonstructural proteins (*i.e.,* RdRp polymerase) that required for self-replication, as a result, the amplified RNA product produced by amplifying the said synthetic RNA fragment in accordance with the present RCR method stated above retains a full-length conformation of the saRNA that is beneficial to mRNA vaccine development.

By virtue of the above features, the present disclosure provides a novel synthetic RNA fragment comprising novel 5'-end and 3'-end RdRp binding sites that recognized and bound by polymerases, thus, enabling an RNA-dependent amplification and replication *in vitro* environment. Further, the 5'-end and 3'-end RdRp-binding sites of the present disclosure form a complementary pair to stabilize the binding of RdRp on the RNA template to initiate RNA synthesis, without being hindered by stem-loop structure. Collectively, the saRNA can be effectively and rapidly synthesized in the RNA-dependent RCR process, by which the thus produced saRNAs retain advantages of high yield, high structural integrity, and high purity, and versatile applications.

### EXAMPLES

### Materials and Methods

### Primers

**Table 1 Primer pairs used in the present application**

| **Primer** | **DNA sequence (5' to 3')** | **SEQ ID NO** |
|---|---|---|
| Viral RdRp, sense | GATATCTAAT ACGACTCACT ATAGGGAGAG GTATGGTACT TGGTAGTT | 1 |
| Viral RdRp, antisense | GACAACAGGT GCGCTCAGGT CCT | 2 |
| pre-miR-302, sense | GATATCTAAT ACGACTCACT ATAGGGAGAT CTGTGGGAAC TAGTTCAGGA AGGTAA | 3 |
| pre-miR-302, antisense | GTTCTCCTAA GCCTGTAGCC AAGAACTGCA CA | 4 |

### Sequences Design

The RNA sequences of 5'- and 3'-end RdRp-binding sites of the present disclosure were designed and derived from SARS-CoV-2 or HCV viruses by computer screening.

### In Vitro RNA Transfection

Isolated messenger RNAs of viral RNA-dependent RNA polymerase (RdRp), and precursor miRNA-302 or S protein of SARS-CoV-2) were mixed at a ratio ranging from 20:1 to 1:20, and dissolved in 0.5 mL of a cell cultivating medium, with addition of 1-50 µL of transfection reagents (in vivo-jetPEI, Westburg, NL) to form a mixture. After incubation for 10-30 minutes, the mixture was then added into another cell cultivating medium containing 50%-60% confluency of the cultivated cells. The medium was reflashed every 12 to 48 hours, depending on cell types.

### Preparation of cDNA templates

Isolated desired RNAs (0.01 ng-10 µg), reverse transcription (RT) primers (0.01-20 nmole), dNTPs, and reverse transcriptase were mixed with the reagent (20-50 µL) of reverse transcription reaction kit (SuperScript III cDNA RT kit, ThermoFisher Scientific, USA) to prepare a reaction mixture by following the manufacturer's indication. The reaction mixture was then incubated at 37-65°C for 1-3 hours, depending on the length and structural complexity of the desired RNA, so as to produce a complementary DNA (cDNA) template thereof. The RT-derived cDNA template (0.01 pg-10 µg) then was mixed with a 20-50 µL of PCR buffer (High-Fidelity PCR master kit, ThermoFisher Scientific, USA) to produce a PCR mixture. The PCR mixture was subjected to reaction cycles composed of denaturation (94°C for 1 minute), annealing (30-58°C for 30 second to 1 minute), and extension (at 72°C for 1-3 minutes), for five to forty cycles, thereby producing an amplified cDNA serving as templates for further studies including an *in vitro* transcription (IVT) as described in published document Li & Ji, Methods in Molecular Biology, vol. 221, 2003, and in U.S. patent application No. 17/648,336, U.S. provisional applications No. 63/302,163 and 63/338,881.

The sequence of RT primer pair used for producing cDNA sequence of viral RNA-dependent RNA polymerase (RdRp) was SEQ ID NO: 1 as listed in Table 1.

### In vitro transcription

The synthesized and amplified cDNA templates were subjected to incorporate the present 5'-end and 3'-end RdRp binding sites onto the cDNA templates by primers specific for PCR (hereinafter, PCR-ready primers), including SEQ ID NOs: 1-4. Specifically, SEQ ID NOs: 1 and 2 were used with cDNA templates containing RdRp gene; and SEQ ID NOs: 3 and 4 were used with cDNA templates containing human pre-miR-302 cluster gene (pre-miR-302). The amplified cDNA templates (0.01 ng-10 µg), 0.1-50 U of viral RdRp and helicase (Abcam, MA, USA/Creative Enzymes, NY), rNTPs, and RNA polymerase including T7, T3, M13 and/or SP6 were mixed in a 1× transcription buffer containing Tris-HCl buffer supplemented with MgCl₂, NaCl, spermidine, optionally added with trimethylglycine (TMG), dimethylsulfoxide (DMSO), and/or 3[N-morpholino]propane sulfonic acid (MOPS), 0.001-10 mM for each, to form a mixture. The mixture was reacted at 30-40°C for 1 to 6 hours, so as to produce the present synthetic RNA fragment for RNA-dependent amplification.

### RNA-dependent RNA cycling reaction (RCR)

The isolated synthetic RNA fragment (0.01 ng-10 µg), a mixture of viral RdRp and helicase (0.1-50 U), and rNTPs were mixed in 1× transcription buffer containing Tris-HCl buffer supplemented with MgCl₂, NaCl, spermidine, TMG, DMSO, and/or MOPS (0.001-10 mM), thereby forming a reaction mixture. The reaction mixture was subjected to RNA-dependent RCR by incubating at 20-45°C for 1-6 hours, thereby producing amplified RNA products.

### Statistics

All data were shown as averages and standard deviations (SD). Mean of each test group and its SD were calculated by AVERAGE and STDEV of Microsoft Excel, respectively. The data was analyzed by One-Way ANOVA. Tukey and Dunnett's t post hoc test were used to identify the significance of data difference in each group by conducting statistical software (SPSS v12.0). *p* <0.05 was considered significant.

### Example 1 Production of the present synthetic RNA fragment containing 5'-end and 3'-end RdRp binding site

The present 5'-end and 3'-end RdRp binding sites were designed and synthesized by methods described in the "Materials and Methods" section. The thus produced 5'-end and 3'-end RdRp binding sites and their RNA sequences are listed in Table 2.

**Table 2 The present 5'-end and 3'-end RdRp binding sites**

| **Sequence (5' to 3')** | |
|---|---|
| 5'-end RdRp binding site | UCUCCUA |
| | UGUGCUA |
| | UCUCCCUA |
| 3'-end RdRp binding site | UAGGAGA |
| | UAGCACA |
| | UAGGGAGA |

Further, cDNA template of viral RdRp and S pike protein of SARS-CoV-2 or precursor miRNA-302 flanked by each of 5'-end RdRp binding site and 3-end RdRp binding site of Table 2 were synthesized, and then subjected to *in vitro* transcription, thereby producing the present synthetic RNA fragment in accordance with procedures described in "Materials and Methods" section.

### Example 2 Producing self-amplifying RNAs by using the synthetic RNA fragment

In this example, whether the present synthetic RNA fragment improves the efficiency of RNA-dependent RCR was evaluated. To this purpose, the synthetic RNA fragment was used as a template for RNA-dependent RCR (amplification), thereby producing self-amplifying RNA (saRNA) in accordance with procedures described in "Materials and Methods" section. The yield of the saRNA was calculated.

It was found that, the RNA-dependent RCR were successfully initiated by the present synthetic RNA fragment, and the saRNA product was produced with a high purity ratio (14/15 to 999/1000). Further, the saRNA product retained the structure integrity, thus would inherit the original ability of self-amplifying when delivered to cells afterwards.

Taken together, the present 5'-end and 3'-end RdRp binding sites are highly complementary to viral RdRp, thus will automatically initiate the direct RNA-mediated amplification in vitro.

It will be understood that the above description of embodiments is given by way of example only and that various modifications may be made by those with ordinary skill in the art. The above specification, examples, and data provide a complete description of the structure and use of exemplary embodiments of the invention. Although various embodiments of the invention have been described above with a certain degree of particularity, or with reference to one or more individual embodiments, those with ordinary skill in the art could make numerous alterations to the disclosed embodiments without departing from the spirit or scope of this invention.

## Claims

1. A synthetic ribonucleic acid (RNA) fragment, comprising:
an RNA template flanked by a 5'-end RNA-dependent RNA polymerase (RdRp) binding site and a 3'-end RdRp binding site, wherein
each of the 5'-end and 3'-end RdRp binding sites individually comprises a polyribonucleotide sequence selected from the group consisting of 5'-USUSCYW-3' and 5'-UAGSRVR-3'.

2. The synthetic RNA fragment of claim 1, wherein the 5'-end RdRp binding site has a polyribonucleotide sequence of 5'-UCUCCUA-3', 5'-UGUGCUA-3', or 5'-UCUCCCU-3' .

3. The synthetic RNA fragment of claim 1, wherein the 3'-end RdRp binding site has a polyribonucleotide sequence of 5'-UAGGAGA-3', 5'-UAGCACA-3', or 5'-UAGGGAGA-3'.

4. The synthetic RNA fragment of claim 1, wherein the RNA template comprises a polyribonucleotide sequence or a hybrid of polyribonucleotide and polydeoxyribonucleotide sequence, wherein the polyribonucleotide sequence is a coding RNA or a non-coding RNA.

5. The synthetic RNA fragment of claim 4, wherein the coding RNA is a messenger RNA (mRNA) that encodes an antigen.

6. The synthetic RNA fragment of claim 5, wherein the antigen is a cancer antigen, a tumor antigen, a bacterial antigen, a viral antigen, a fungal antigen, a parasitic antigen, or a combination thereof.

7. The synthetic RNA fragment of claim 6, wherein the tumor antigen is selected from the group consisting of a neoantigen, a tumor-derived lysate, an alpha-fetoprotein (AFP), a carcinoembryonic antigen (CEA), a mucin protein, an epithelial tumor antigen (ETA), a tyrosinase, a melanoma-associated antigen (MAGE), a RAS protein, and a tumor suppressor protein.

8. The synthetic RNA fragment of claim 6, wherein the bacterial antigen is derived from a bacterial species selected from the group consisting of *Actinomyces, Aeromonas, Arthrobacter, Bacillus, Bacteroides, Bordetella, Borrelia, Brucella, Campylobacter, Chlamydia, Citrobacter, Clostridium, Corynebacterium, Escherichia, Enterobacter, Gardnerella, Helicobacter, Haemophilus, Klebsiella, Legionella, Listeria, Mycobacterium, Neisseria, Nocardia, Pasteurella, Proteus, Pseudomonas, Ureaplasma, Salmonella, Shigella, Spirillum, Spirochaeta, Staphylococcus, Streptobacillus, Streptococcus, Streptomyces, Treponema,* and *Yersinia.*

9. The synthetic RNA fragment of claim 6, wherein the viral antigen is derived from a viral species selected from the group consisting of *Adenovirus, Alphacoronavirus, Betacoronavirus, Cytomegalovirus, Deltainfluenzavirus, Deltacoronavirus, Gammacoronavirus, Hepacivirus, Hepatovirus, Influenza A virus, Influenza B virus, Influenza C virus, Influenza D virus, Lentivirus, Letovirus, Lymphocryptovirus, Orthopneumovirus, Orthohepadnavirus, Orthopoxvirus, Papillomavirus, Quaranjavirus, Rotavirus, Simplexvirus,* and *Varicellovirus.*

10. The synthetic RNA fragment of claim 9, wherein the viral antigen is derived from a spike protein of *Betacoronavirus.*

11. The synthetic RNA fragment of claim 6, wherein the fungal antigen is derived from a fungal species that causes a fungal infection selected from the group consisting of aspergillosis, blastomycosis, candidiasis, chromoblastomycosis, cryptococcosis, histoplasmosis, mycetoma, paracoccidioidomycosis, ringworm and tinea versicolor.

12. The synthetic RNA fragment of claim 6, wherein the parasitic antigen is derived from a parasite species that causes a parasitic infection selected from the group consisting of African trypanosomiasis, amebiasis, Chagas disease, echinococcosis, fascioliasis, hookworm disease, hymenolepis, leishmaniasis, neurocysticercosis, onchocerciasis, *Plasmodium* infection, paragonimiasis, *Pneumocystis* pneumonia (PCP), schistosomiasis, trichomoniasis, taeniasis, and trichuriasis.

13. The synthetic RNA fragment of claim 4, wherein the non-coding RNA is a small interfering RNA (siRNA), a ribosomal RNA (rRNA), a transfer RNA (tRNA), a microRNA (miRNA), or an aptamer.

14. The synthetic RNA fragment of claim 13, wherein the miRNA is a precursor miRNA or a mature miRNA.

15. The synthetic RNA fragment of claim 14, wherein the non-coding RNA is derived from the precursor miRNA-302.

16. The synthetic RNA fragment of claim 1, wherein the RNA template comprises a polyribonucleotide sequence encoding a transcript of a nonstructural protein.

17. The synthetic RNA fragment of claim 16, wherein the nonstructural protein is an RNA-dependent RNA polymerase (RdRp).

18. A method for producing an amplified RNA product *in vitro* comprising amplifying the synthetic RNA fragment of claim 1 via an RNA cycling reaction (RCR), thereby producing the amplified RNA product transcribed from the RNA template of the synthetic RNA fragment of claim 1.

19. The method of claim 18, wherein the RNA template of the synthetic RNA fragment comprises a polyribonucleotide sequence or a hybrid of polyribonucleotide and polydeoxyribonucleotide sequence, and the polyribonucleotide sequence encodes a transcript of a nonstructural protein.

20. The method of claim 18, further comprising adding a five-prime cap (5'cap) and a poly(A) tail to the amplified RNA product.

21. The method of claim 18, wherein the amplified RNA product is a self-amplifying RNA (saRNA).
